(19) Europäisches Patentamt
European Patent Office
Office européen des brevets

(11) **EP 0 676 194 B2**

(12) **NOUVEAU FASCICULE DE BREVET EUROPEEN**
Après la procédure d'opposition

(45) Date de publication et mention de la décision concernant l'opposition:
**07.01.2015 Bulletin 2015/02**

(45) Mention de la délivrance du brevet:
**14.11.2001 Bulletin 2001/46**

(21) Numéro de dépôt: **95400694.6**

(22) Date de dépôt: **29.03.1995**

(51) Int Cl.:
***A61K 31/60*** *(2006.01)*

(54) **Compositions cosmétiques ou dermatologiques contenant un alpha-hydroxyacide de l'acide salicylique et un retinoide**

Kosmetische oder dermatologische Zusammensetzung, die ein alpha-Hydroxysäure, Salicylsäure und ein Retinoid enthalten

Cosmetic or dermatological compositions comprising an alpha-hydroxy acid, salicylic acid and a retinoid

(84) Etats contractants désignés:
**AT BE CH DE DK ES FR GB GR IE IT LI LU NL PT SE**

(30) Priorité: **01.04.1994 FR 9403873**

(43) Date de publication de la demande:
**11.10.1995 Bulletin 1995/41**

(73) Titulaire: **Beiersdorf AG**
**20245 Hamburg (DE)**

(72) Inventeurs:
• **Bobier-Rival, Carinne**
  **F-95000 Cergy (FR)**
• **Fournier, Odile**
  **F-92500 Rueil Malmaison (FR)**
• **Fructus, Alain**
  **F-92400 Courbevoie (FR)**

(56) Documents cités:
EP-A1- 0 610 511    EP-A2- 0 273 202
EP-A2- 0 508 324    WO-A1-94/01083
WO-A1-95/04537    FR-A- 2 310 767

GB-A- 2 188 233    US-A- 4 216 224
US-A- 5 153 230

• MICROMEDEX, INC., DENVER, COLORADO, VOL. 82, FILE MARTINDALE, THE EXTRA PHARMACOPOEIA., XP002024677
• MICROMEDEX, INC., DENVER, COLORADO, VOL. 82, FILE MARTINDALE, THE EXTRA PHARMACOPOEIA., XP002024678
• MICROMEDEX, INC., DENVER, COLORADO, VOL. 82, FILE MARTINDALE, THE EXTRA PHARMACOPOEIA., XP002024679
• MICROMEDEX, INC., DENVER, COLORADO, VOL. 82, FILE MARTINDALE, THE EXTRA PHARMACOPOEIA., XP002024680
• MICROMEDEX, INC., DENVER, COLORADO, VOL. 82, FILE MARTINDALE, THE EXTRA PHARMACOPOEIA., XP002024681
• Schreiben des Patentanmelders "The Boots Company Nottingham" zur Darlegung des synergistichen Effektes des Patentgegenstandes vom 24.03.2000
• E. MUTSCHLER ET AL.: 'Arzneimittelwirkungen', vol. 5, 1986, WISSENSCHAFTLICHE VERLAGSGESELLSCHAFT MBH, STUTTGART pages 549 - 552
• J. FALBE ET AL.: 'Römpp Chemie Lexikon', vol. 9, 1992, GEORG THIEME VERLAG, STUTTGART - NEW YORK

**EP 0 676 194 B2**

**Description**

La présente invention concerne de nouvelles compositions cosmétiques ou dermatologiques qui contiennent au minimum trois principes actifs et leur préparation.

L'utilisation par les dermatologues d'alpha-hydroxy-acides à des doses de 30 à 70 % est un usage très ancien pour réduire les cicatrices profondes. Par exemple, on a proposé l'usage de l'acide lactique pour le traitement de certaines pathologies de la peau et pour le contrôle de la kératinisation. Il est connu d'utiliser de nombreux types d'alpha-hydroxy-acides pour traiter les hyperkératinisations comme les cals, les pellicules, la peau craquelée, l'ichtyose, l'acné, la peau très sèche, les kératoses actiniques, les taches de vieillesse.

Par exemple, dans le brevet US 3 878 537, les acides glycolique, citrique, malique, tartronique, tartarique, glucuronique, pyruvique, 2-hydroxybutyrique, 3-hydroxybutyrique et l'acide lactique sont décrits pour traiter l'ichtyose. Le pH préconisé des préparations est de 3,5 à 7,5. La teneur en acide varie de 1 à 20 % et la composition peut être un mélange d'eau et d'alcool ou une pommade appropriée.

Le mode d'action des alpha-hydroxy-acides semble se situer au niveau des couches profondes du stratum cornéum. Ce mécanisme n'est pas complètement élucidé mais il semble qu'il se passe en deux temps, d'abord le blocage des sites de liaisons ioniques entre les cornéocyties et ensuite le blocage des systèmes enzymatiques de type sulfatases et phosphorylases (voir Ichihara, Greenberg, J. Biol. Chem. 225, 945-958 (1957)).

L'utilisation de l'acide salicylique est très connu en dermatologie pour son effet kératolytique. De plus, l'association alpha-hydroxy-acides et acide salicylique est mentionné dans le brevet DD 274357.

L'acide salicylique est un kératolytique puissant par action directe (solubilisation) sur la kératine des couches les plus superficielles de la couche cornée.

L'utilisation des rétinoïdes est bien connue en dermatologie et en cosmétologie pour les traitements de l'acné, des kératoses actiniques et des rides dues au vieillissement. Il semblerait que les rétinoïdes détruisent les desmosomes des kératinocytes et provoquent donc une desquamation.

Par ailleurs, les rétinoïdes sont reconnus par des récepteurs du noyau cellulaire et agissent sur la régulation de la différenciation des kératinocytes en la stimulant.

L'association de vitamine A et d'alpha-hydroxy-acides est décrite par le brevet US 5153230.

Ainsi, les utilisations d'alpha-hydroxy-acides associés à l'acide salicylique ou à la vitamine A sont connus dans le traitement des troubles de la kératinisation. Les pH utilisés dans ces diverses préparations sont de 3,5 à 7,5 plus rarement de 3,0 à 6,0.

Avec les compositions cosmétiques connues, les effets secondaires comme le picotement, le rougissement ou les sensations de brûlures ne sont pas négligeables.

Dès que les taux d'alpha-hydroxy-acides sont au-dessus de 20 %, il y a nécessité de rincer après un certain temps d'application ce qui rend ces compositions presque impropres à un usage cosmétique et les réserve à un usage sous contrôle médical.

En poursuivant les études sur les produits précités, la demanderesse vient de trouver de façon tout à fait inattendue que l'utilisation d'une composition renfermant au minimum trois principes actifs, dont 3 à 20 % en poids d'un ou plusieurs alpha-hydroxy-acides, 0,1 à 5 % en poids de l'acide salicylique ou 0,2 à 10 % en poids d'un ester ou un melange d'esters de cet acide et 0,02 à 2 % en poids d'au moins un retinoïde permet de combiner les effets desquamants, hydratants des alpha-hydroxy-acides, l'effet kératolytique de l'acide salicylique et les effets desquamants et stimulateurs de différenciation kératinocitaire des rétinoïdes. L'invention a ainsi pour objet une telle composition cosmétique ou dermatologique. L'application sur la peau d'une telle association permet de traiter les problèmes liés au vieillissement par effet desquamant doux et régénération de l'épiderme. D'autres applications de l'invention concernent les problèmes liés à une hyperkératinisation comme l'acné, les kératoses actiniques, les tâches de vieillesse, la peau sèche et très sèche.

L'association précédemment décrite, en permettant des actions différentes et complémentaires, ne nécessite pas des concentrations aussi élevées que dans le cas de produit seul ou en association à deux, et a donc une meilleure tolérance sur la peau.

Les compositions cosmétiques ou dermatologiques selon l'invention contiennent préférentiellement :

4 à 15 %, d'un ou plusieurs alpha-hydroxy-acides,
0,3 à 4 %, d'acide salicylique ou 0,6 à 8% d'un ester ou d'un mélange d'esters de l'acide salicylique et
0,04 à 1 %, de rétinoïde.

Les pourcentages sont exprimés en % du poids total de la composition.

Les alpha-hydroxy-acides sont préférentiellement choisis parmi les acides lactiques, glycolique, malique, tartronique, tartarique, glucuronique, pyruvique, 2-hydroxyisobutyrique, 3-hydroxybutyrique, citrique, galacturonique, mandélique, mucique β-phényllactique, β-phénylpyruvique, saccharique, α-hydroxybutyrique, α-hydroxyisobutyrique, α-hydroxyisocaproïque, α-hydroxyisovalérique, atrolactique, galactanique, pantoïque, glycérique, isocitrique, dihydroxymaléïque, dihydroxytartarique, dihydroxyfumarique, benzylformique. Les acides lactiques et glycoliques sont préférés. Préférentiellement, on utilise la combinaison de deux acides.

En outre, on peut également utiliser des esters d'alpha-hydroxy-acides qui ont pour effet de libérer plus lentement les alpha-hydroxy-acides dans la peau.

Parmi ces esters, on peut citer notamment le cosmacol ETL® (Di C14-C15 alkyl tartrate), le cosmacol ECL® (Tri C14-C15 alkyl citrate), le cosmacol ELI® C12-C13 alkyl lactate), le cosmacol FOI® (C12-C13 alkyl octanoate), le cosmacol EMI® (Di C12-C13 alkyl malate), le cosmacol ECI® (Tri C12-C13 alkyl citrate), le cosmacol ETI® (Di C12-C13 alkyl tartrate).

Les dosages de ces esters, seuls ou en mélange, varient de 2 à 15% et préférentiellement de 4 à 10% du poids total de la composition.

Parmi les esters préférés de l'acide salicylique, que l'on peut utiliser seul ou en mélange, on peut citer notamment l'isodécyl salicylate, le tridécyl salicylate ou encore l'isopropylbenzyl salicylate.

Les rétinoïdes sont choisis parmi le rétinol, l'acétate de rétinol, le palmitate de rétinol, l'acide rétinoïque 13-cis, l'acide rétinoïque tout trans, les rétinoylphospholipides comme le rétinoyl 13-cis phosphatidyléthanol ou le rétinoyl tout trans phosphatidyléthanol.

Les rétinoïdes peuvent se présenter sous forme pure ou en solution dans une huile ou un alcool. Ils peuvent être sous la forme de liposomes ou de vésicules huileux entourés d'une couche de phospholipides, de cholestérol et d'acides gras (comme les LIPOMICRONS® de la Société SEDERMA ou NANOFUTURE® du Pr WEBER ou NANO-COS® de la Société INDUCHEM). Ces différentes formes ont pour objet de protéger les rétinoïdes de l'oxydation et de la dégradation chimique pendant la fabrication du produit complet et pendant le stockage du produit complet avant utilisation. Elles ont également pour objet de faciliter la pénétration des rétinoïdes dans la peau.

Les rétinoïdes peuvent également être associés à des cristaux liquides (comme les LICRITHERM® de la Société MERCK ou les cristaux liquides de la Société HALLCREST). Ces cristaux liquides ont également pour but de protéger les rétinoïdes et de faciliter leur pénétration dans la peau.

L'association décrite dans la présente invention se présente préférentiellement sous forme de gel aqueux, d'émulsion simple ou d'émulsion multiple, mais d'autres formes sont également possibles.

Dans le cas d'une émulsion simple, on choisira préférentiellement le sens eau dans huile. On a trouvé que dans le choix d'une émulsion eau dans huile, le pH de la solution aqueuse contenant le ou les alpha-hydroxy-acides et l'acide salicylique ou un ou plusieurs de ses esters, le pH de cette solution pouvait être très bas, spécialement entre 1,8 et 2,5, sans qu'il n'y ait d'effets désagréables après application sur la peau. Ce pH très bas permet encore de réduire la teneur en acides pour une efficacité équivalente.

Dans le cas d'une émulsion multiple, on choisira le sens eau dans huile dans eau. Le mélange du ou des acides alpha-hydroxy-acides et de l'acide salicylique d'un ester ou d'un mélange de ses esters sera dans la phase aqueuse interne avec un pH compris préférentiellement entre 1,8 et 2,5.

L'invention a également pour objet le traitement des kératoses actiniques, en ajoutant au mélange précédemment décrit de l'acide nordihydroguaiarétique (ou NDGA) ou un ester de NDGA comme le NDGA phosphatinyl éthanol (acide NDGA greffé en position 2 d'un phosphatinyl éthanol) et l'invention a donc particulièrement pour objet de telles compositions. La concentration en NDGA ou en ester de NDGA peut être de 0,05 à 10 % par rapport à la composition totale, préférentiellement de 0,1 à 5 %.

La composition cosmétique comprenant des acides alpha-hydroxylés, de l'acide salicylique ou un ester ou un mélange de ses esters, un rétinoïde et du NDGA, par ses effets desquamants, kératolytique et régénérant, répare particulièrement bien la peau atteinte de kératose actinique.

La présente invention a également pour objet le traitement des taches de vieillissement par ajout au mélange décrit précédemment d'un ou plusieurs agents connus pour leurs effets anti-taches et l'invention a aussi plus particulièrement pour objet de telles compositions.

Les agents connus pour leurs effets anti-taches sont choisis par exemple parmi l'acide kojique (0,01 à 2 %), le LICORICE PT® de la Société NIKKO (0,01 à 3 %),

le calcium Panthotéine sulfonate (0,1 à 5 %),
le méthiosilane C+® de la Société EXYMOL (de 1 à 10 %),
l'ARBUTINE (principe actif de la Bucerole) (de 0,1 à 5 %) ou sa forme liposomée (de 1 à 10 %),
le MELAWHITE® de la Société PENTAPHARM (de 0,5 à 5 %),
l'UNIMONTAN® de la Société INDUCHEM (de 1 à 10 %),
le CELL SEED EXA® de la Société SPCI (0,01 à 1 %).

La présente invention a également pour objet le traitement de l'acné. En effet, l'association précédemment décrite permet, par son action desquamante et kératolytique, de désobstruer les orifices sébacés dont la kératinisation excessive est la première étape dans l'étiologie des comédons, des micro-kystes puis des boutons d'acné. Une ou plusieurs substances actives adaptées au traitement de l'acné peuvent être ajoutées au mélange précédemment décrit, notamment des agents anti-bactériens, des agents régulateurs de sébum, des anti-inflammatoires, des anti-lipases, des absorbeurs de sébum ou d'acide gras, des astringents et l'invention a aussi particulièrement pour objet de telles compositions.

Ces agents anti-acné sont plus particulièrement choisis parmi :

l'acétate d'oléyle (0,5 à 5 %) anti-lipase,
le Biolysat de Hafnia (0,01 à 1 %) anti-inflammatoire, (comme décrit par FR-B 8406559)
le CITRICINAL® (0,01 à 1 %) bactéricide,
la chlorhexidine hibitane (0,2 à 3 %) bactéricide,
l'huile essentielle de genévrier (0,01 à 2 %) bactéricide, l'undécylénate de sodium (0,2 à 3 %) bactéricide,
l'acétate de zinc (0,2 à 2 %) bactéricide,
le trichlorocarban (0,05 à 2 %) bactéricide,
l'ALCLOXA® de la Société HOECHST (0,2 à 3 %) astringent,
le MICROPEARL® de la Société SEPPIC (1 à 10 %) absorbant de sébum,
le TAKALOPHANE® de la Société NIKKO (0,5 à 5 %) absorbant d'acide gras,
le kaolin (1 à 10 %) absorbant de sébum,
le stéarylglycyrrhétinate (0,2 à 2 %) anti-inflammatoire,
l'acide glycyrrhétinique (0,1 à 2 %) anti-inflammatoire,
le LIPACIDE PVS® de la Société SEPPIC (0,5 à 5 %) anti-inflammatoire,
l'extrait essentiel d'huile de sésame de la Société EXPANSCIENCE (0,2 à 5 %) anti-inflammatoire,
l'huile de maïs peroxydée EPALINE 100® de la Société CARILENE (1 à 10 %) anti-inflammatoire,
le DEFFATED RICE BRAN PF-60® de la Société TSUNO (0,5 à 10 %) absorbant de sébum,
le SHISO extract (Jan Dekker, France)(0,2 à 3 %) bactéricide,
le SUNPHENON® (Jan Dekker, France)(0,2 à 2 %) bactéricide,
la PHLOROGINE® de la Société SECMA (0,2 à 5 %) régulateur de sébum.

L'invention a tout particulièrement pour objet une composition renfermant :

4 à 15% en poids d'acide lactique
0,3 à 4% en poids d'acide salicylique ou 0,6 à 8% en poids d'un de ses esters
et 0,04 à 1% en poids de palmitate de vitamine A.

L'invention concerne aussi un procédé de préparation d'une composition cosmétique ou dermatologique telle que définie précédemment caractérisé en ce que l'on prépare une émulsion de type eau dans huile (silicone), la phase aqueuse contenant les alpha-hydroxy-acides et que l'on ajoute à cette émulsion, l'acide salicylique ou un ester ou un mélange de ses esters et au moins un rétinoïde.

L'invention a également pour objet le traitement du vieillissement de la peau par ajout au mélange précité, de molécules actives comme des produits anti-élastase (par exemple un extrait de Klebsiella pneumonia), des produits anti-radicalaires (par exemple les extraits de Sily marine mélangés ou non à du tocophérol), des céramides naturelles ou synthétiques (comme la céramide 2) ou des équivalents céramides (comme la céramide HO3 de la Société SEDERMA).

**EXEMPLE 1 : Préparation d'une crème de soin destinée à lutter contre le vieillissement par action desquamante et régénératrice**

On prépare la phase grasse suivante :

- Stéarate d'isocétyle          3 g
- Sorbitan sesquioléate          0,8 g
- Huile de ricin hydrogénée          0,9 g
- Huile de jojoba          1 g
- PEG-45 (dodécyl glycolcopolymer) Elfacos ST 9® de la Société AKZO          2 g
- Cyclométhicone/diméthicone copolyol          9 g
- Silicone volatil          4 g

Cette phase grasse est chauffée à 60°C environ jusqu'à complète homogénéité.
On prépare, par ailleurs, la phase aqueuse suivante :

- Eau        45,98 g
- Solution d'acide lactique à 40 % obtenu par fermentation de jus de canne de pomme et de citron et additionné de 2 % d'extrait de thé vert        10 g
- Acide glycolique à 55 %        1 g
- Chlorure de sodium        0,8 g
- Tétrasodium EDTA        0,05 g
- Polysaccharide obtenu par fermentation (sclérosium gum)        0,6 g
- Glycérine        3 g
- Propylène glycol        3 g
- PEG-400        1,5 g
- Méthyl paraben        0,25 g
- Propyl paraben        0,15 g
- O-Cymen-5-OL        0,1 g

Ces produits sont mélangés jusqu'à dissolution complète. Le pH de la phase aqueuse est règlé à 2,3 à l'aide de 0,07 g environ de soude pure.

La phase aqueuse est ensuite ajoutée sous agitation vigoureuse à la phase grasse à une température de 60°C. L'émulsion eau/silicone se forme immédiatement. L'agitation est poursuivie pendant environ 1/2 heure en diminuant l'agitation et en laissant la température baisser jusqu'à la température ambiante.

On ajoute ensuite, sous agitation réduite, l'acide salicylique préalablement dissous dans l'éthanol :

- Acide salicylique        0,5 g
- Ethanol à 95°        3 g

On ajoute ensuite une suspension de Lipomicron® de palmitate de vitamine A (6 g) (correspondant à 0,1 g du rétinoïde), un parfum (0,3 g) et enfin 3 g de talc de Luzenac, sous agitation modérée.

Cette émulsion a une viscosité de 24 000 cP (prise au viscosimètre Brookfield RVT, 24 h après fabrication à la température d'environ 20°C). Elle est stable à la centrifugation (15 min. à 5.000 tr/min.) et plus de 3 mois à la température de 42°C.

Cette émulsion est appelée préparation I.

En opérant comme à l'exemple 1, on a préparé les compositions suivantes :

### EXEMPLE 2 :

On prépare l'émulsion eau/huile suivante :

- Phase grasse :
- ABIL EM90® (cétyl diméthicone copolyol)        4 g
- Polysorbate 20        0,2 g
- Huile de jojoba        1 g
- ELF Acos ST 45®        0,6 g
- C12-15 alkylbenzoate        5 g
- Cosmacol ECI®        5 g
- Palmitate de Vitamine A        0,12g
- Huile de Silicone        2 g
- Isodécyl salicylate        1,5 g

- Phase aqueuse :
- Glycérine        3 g
- Sclerosium gum        0,2 g
- Chlorure de sodium        0,8 g
- Acide lactique        5 g
- Acide glycolique        1 g
- Soude caustique        Q.S. pH        2,2
- Conservateurs        0,5 g
- Parfum        0,2 g
- Eau        Q.S.        100 g

**EXEMPLE 3 :**

Emulsion eau/huile avec un autre type d'émulsionnant :

- <u>Phase grasse</u> :
- Arlacel 1689®        3 g
- Triglycéride caprylique/caprique        4 g
- Isohexadécane        8 g
- Huile de vaseline        5 g
- Tridecyl salicylate        2 g
- Isopropyl benzyl salicylate        2 g
- Retinoyl 13-cis phosphatidylethanol        0,5 g


- <u>Phase aqueuse</u> :
- Glycérine        4 g
- Sulfate de magnésium        0,5 g
- Acide lactique        4 g
- Acide glycolique        2 g
- Cosmacol ECL®        2 g
- Conservateurs        0,5 g
- Parfum        0,3 g
- Eau        Q.S.        100 g

**EXEMPLE 4 :**

Emulsion gel crème huile dans eau :

- <u>Phase grasse</u> :
- Huile de jojoba        5 g
- Di-isocetyl cyclohexane        5 g
- Huile de silicone        2 g
- Acétate de rétinol        1 g
- Parfum        0,5 g

- <u>Phase aqueuse</u> :
- SEPIGEL 305®        3 g
- Isononanoate d'isononyle        5 g
- Huile de silicone        2 g
- Acide glycolique        2 g
- Acide malique        3 g
- Conservateurs        0,5 g
- Alcool 96        5 g
- Acide salicylique        0,5 g
- Penederm PP15®        2 g
- Eau        Q.S.        100 g

**Application de la composition cosmétique**

L'étude a pour but de montrer la supériorité de la composition selon l'invention (préparation I) par rapport aux autres mélanges (préparations II, III, IV, V et VI), sur la vitesse de desquamation de cellules du stratum corneum par la méthode de la dihydroxyacétone (DHA).

A) <u>Méthodologie</u>

L'étude a été menée chez 5 sujets sains, caucasiens, dont 4 femmes et 1 homme, âgés de 25 à 50 ans.

**Produits :**

5 produits ont été testés :

Préparation I     Produit comme décrit dans l'exemple 1
Préparation II    Placébo,
Préparation III   Préparation I, mais sans acide lactique,
Préparation IV    Préparation I, mais sans acide salicylique,
Préparation V     Préparation I, mais sans lipomicron de palmitate de vitamine A.

Le pH de la phase aqueuse a été ajusté à 2,2 pour tous les produits, sauf le placébo (pH = 5,7). Le produit placébo correspond à la préparation dans l'exemple 1, sans $\alpha$-hydroxy acides, sans acide salicylique et sans rétinoïde.

**Mode et lieu d'application :**

Trois zones de 10 cm$^2$ chacune ont été délimitées sur la face antérieure de chaque avant-bras.
Deux applications d'une crème huile/eau contenant 10 % de la dihydroxyacétone ont été faites à 24 heures d'intervalle, afin d'obtenir une coloration nette et uniforme.
Une quantité définie (0,02 ml, soit 2 mg/cm$^2$) des six produits a été appliquée uniformément en fine couche à l'aide d'un doigtier avec un massage de quelques secondes pendant 9 jours, à raison de deux applications/jour. Les produits ont été appliqués avec une seringue Terumo Ribbon Pack de 1 ml.

**Mesures :**

Les mesures ont été effectuées aux temps suivants :

T0 = avant application de la DHA,
T1 = 48 heures après les deux application de la DHA,
T2, T3, T4, T5, T6, T7, T8 = après 1, 2, 3, 4, 7, 8 et 9 jours applications des produits à étudier.

**Procédure technique :**

La détermination de la couleur de la peau est faite à l'aide d'un chromamètre (par exemple Chromamètre CR-200 MINOLTA) équipé d'une lampe à arc xénon pulsé.
Le principe de l'appareil est basé sur la mesure de la lumière réfléchie par la surface cutanée. Les cellules du chromamètre convertissent la lumière reçue en courant dont l'intensité est proportionnelle à l'éclat de la lumière.
Le courant est ensuite changé en signal digital lui-même traité par un microprocesseur qui détermine les valeurs tristimulus de la surface dans l'espace de couleur sélectionné (ici espace L* a* b*) (voir Minolta "Analyse de couleurs - contrôle de couleurs : de la perception à l'instrumentation" Janvier 1989).
La couleur telle qu'on la perçoit a trois dimensions : teinte, saturation et clarté.
La chromaticité inclut la teinte et la sutaration ; elle est spécifiée par 2 coordonnées chromatique a* et b*.
Chaque valeur correspond à la moyenne des 3 mesures.

B) <u>Résultats</u>

Ils sont consignés sur des feuilles d'observations, une par participant.
Le paramètre étudié au cours de l'étude est la composante jaune b*. Ce paramètre a été choisi en raison de sa décroissance linéaire au cours du temps, directement liée à une perte en intensité de la coloration obtenue après application de l'autobronzant (voir Pierard et Pierard - Franchimont, Dermatology, 1992, 663/348).
Le calcul du pourcentage de diminution du paramètre b* par rapport à T1 est fait selon la formule suivante :

$$\% \ Tx = (T1-Tx) \ / \ (T1-T0) \ x \ 100$$

où Tx représente consécutivement T2, T3, T4, T5, T6, T7 et T8.
Les valeurs moyennes (sur 5 sujets) sont rassemblées dans le tableau suivant :

| % dim. b∗/temps | Prép. I | Prép. II | Prép. III | Prép. IV | Prép. V |
|---|---|---|---|---|---|
| % T2 | 11 | 4 | 6 | 10 | 12 |
| % T3 | 18 | 11 | 14 | 16 | 17 |
| % T4 | 26 | 15 | 18 | 21 | 23 |
| % T5 | 34 | 22 | 25 | 25 | 32 |
| % T6 | 60 | 31 | 43 | 38 | 45 |
| % T7 | 71 | 36 | 61 | 44 | 59 |
| % T8 | 81 | 52 | 74 | 59 | 75 |

**Analyse des résultats**

Après application de la crème contenant 10 % de la DHA (T1), on enregistre une augmentation des valeurs du paramètre b* par rapport au départ (T0). Ensuite les valeurs de b* suivent une décroissance progressive pendant les 9 jours d'application pour tous les produits testés. Cette décroissance est exprimée en pourcentage par rapport à T1 et elle est supérieure pour la préparation I (produit complet) par rapport aux autres préparations (II, III, IV et V) contenant le mélange de 2 des principes actifs seulement.

L'avantage de la préparation selon l'invention devient plus fort après plusieurs semaines d'application.

De plus, le produit a une très bonne innocuité.

**Revendications**

1. Composition cosmétique ou dermatologique renfermant un ou plusieurs alpha-hydroxy-acides, de l'acide salicylique ou un ester ou un mélange d'esters de cet acide et au moins un rétinoïde, **caractérisée en ce qu'**elle renferme :

   3 à 20 % en poids d'alpha-hydroxy-acides,
   0,1 à 5 % en poids d'acide salicylique ou 0,2 à 10% en poids d'un ester ou d'un mélange d'esters de cet acide et
   0,02 à 2 % en poids de rétinoïde.

2. Composition cosmétique ou dermatologique selon la revendication 1 renfermant un ou plusieurs alpha-hydroxy-acides, de l'acide salicylique et au moins un rétinoïde.

3. Composition selon une des revendications 1 à 2, **caractérisée en ce qu'**elle renferme aussi de l'acide nordihydroguaiarétique ou un ester de cet acide.

4. Composition selon une des revendications 1 à 3, **caractérisée en ce qu'**elle renferme aussi un ou plusieurs agents anti-taches.

5. Composition selon une des revendications 1 à 4, **caractérisée en ce qu'**elle renferme aussi un composé anti-inflammatoire et/ou anti-bactérien et/ou anti-lipase et/ou absorbeur de sébum ou d'acide gras.

6. Composition selon une des revendications 1 à 5, **caractérisée en ce qu'**elle renferme

   4 à 15 % en poids d'acide lactique,
   0,3 à 4 % en poids d'acide salicylique ou 0,6 à 8% en poids d'un ester ou d'un mélange d'esters de cet acide et
   0,04 à 1 % en poids de palmitate de vitamine A.

7. Procédé de préparation d'une composition cosmétique ou dermatologique telle que définie aux revendications 1 à 6, **caractérisé en ce que** l'on prépare une émulsion de type eau dans silicone, la phase aqueuse contenant les alpha-hydroxy-acides, et que l'on ajoute à cette émulsion l'acide salicylique ou un ester ou un mélange d'esters de cet acide et au moins un rétinoïde.

8. Application à titre de produit cosmétique d'une composition telle que définie aux revendications 1 à 6.

**9.** Méthode de traitement cosmétique, **caractérisée en ce qu'**on utilise une composition cosmétique telle que définie aux revendications 1 à 6.

**Patentansprüche**

**1.** Kosmetische oder dermatologische Zusammensetzung, bestehend aus einer oder mehreren $\alpha$-Hydroxysäuren, Salicylsäure oder einem Ester oder einer Mischung aus Estern dieser Säure und mindestens einem Retinoid, **dadurch gekennzeichnet, daß** sie aus folgendem besteht:

3 bis 20 Gew.-% $\alpha$-Hydroxysäuren,
0,1 bis 5 Gew.-% Salicylsäure oder 0,2 bis 10 Gew.-% eines Esters oder einer Mischung aus Estern dieser Säure und
0,02 bis 2 Gew.-% Retinoid.

**2.** Kosmetische oder dermatologische Zusammensetzung gemäß Anspruch 1, bestehend aus einer oder mehreren $\alpha$-Hydroxysäuren, Salicylsäure und mindestens einem Retinoid.

**3.** Zusammensetzung gemäß einem der Ansprüche 1 bis 2, **dadurch gekennzeichnet, daß** sie ferner aus Nordihydroguajaretsäure oder aus einem Ester dieser Säure besteht.

**4.** Zusammensetzung gemäß einem der Ansprüche 1 bis 3, **dadurch gekennzeichnet, daß** sie ferner aus einem oder mehreren Mitteln gegen Altersflecken besteht.

**5.** Zusammensetzung gemäß einem der Ansprüche 1 bis 4, **dadurch gekennzeichnet, daß** sie ferner aus einer entzündungshemmenden und/oder antibakteriellen Verbindung und/oder Verbindung gegen Lipasen und/oder Verbindung zur Absorption von Talg oder Fettsäure besteht.

**6.** Zusammensetzung gemäß einem der Ansprüche 1 bis 5, **dadurch gekennzeichnet, daß** sie aus folgendem besteht:

4 bis 15 Gew.-% Milchsäure,
0,3 bis 4 Gew.-% Salicylsäure oder 0,6 bis 8 Gew.-% eines Esters oder einer Mischung aus Estern dieser Säure und
0,04 bis 1 Gew.-% Vitamin-A-Palmitat.

**7.** Verfahren zur Herstellung einer kosmetischen oder dermatologischen Zusammensetzung gemäß Ansprüchen 1 bis 6, **dadurch gekennzeichnet, daß** eine Wasser-in-Silikon-Emulsion zubereitet wird, wobei die wäßrige Phase $\alpha$-Hydroxysäuren enthält, und daß dieser Emulsion Salicylsäure oder ein Ester oder ein Gemisch aus Estern dieser Säure und mindestens ein Retinoid zugeführt wird.

**8.** Anwendung einer Zusammensetzung gemäß Ansprüchen 1 bis 7 als kosmetisches Produkt.

**9.** Verfahren zur kosmetischen Behandlung, **dadurch gekennzeichnet, daß** eine kosmetische Zusammensetzung gemäß Ansprüchen 1 bis 6 verwendet wird.

**Claims**

**1.** Cosmetic or dermatological composition containing one or more alpha-hydroxy acids, salicylic acid or an ester or a mixture of esters of this acid and at least one retinoid, **characterized in that** it contains:

3% to 20% by weight of alpha-hydroxy acids,
0.1% to 5% by weight of salicylic acid or 0.2% to 10% by weight of an ester or a mixture of esters of this acid, and
0.02% to 2% by weight of a retinoid.

**2.** Cosmetic or dermatological composition according to Claim 1, containing one or more alpha-hydroxy acids, salicylic acid and at least one retinoid.

3. Composition according to either of Claims 1 and 2, **characterized in that** it also contains nordihydroguaiaretic acid or an ester of this acid.

4. Composition according to one of Claims 1 to 3, **characterized in that** it also contains one or more anti-blemish agents.

5. Composition according to one of Claims 1 to 4, **characterized in that** it also contains an antiinflammatory and/or antibacterial and/or anti-lipase and/or sebum-absorbing or fatty-acid-absorbing compound.

6. Composition according to one of Claims 1 to 5, **characterized in that** it contains:

   4% to 15% by weight of lactic acid,
   0.3% to 4% by weight of salicylic acid or 0.6% to 8% by weight of an ester or a mixture of esters of this acid, and
   0.04% to 1% by weight of vitamin A palmitate.

7. Process for preparing a cosmetic or dermatological composition as defined in Claims 1 to 6, **characterized in that** an emulsion of water-in-silicone type is prepared, the aqueous phase containing the alpha-hydroxy acids, and **in that** salicylic acid or an ester or a mixture of esters of this acid and at least one retinoid are added to this emulsion.

8. Use, as a cosmetic product, of a composition as defined in Claims 1 to 6.

9. Cosmetic treatment method, **characterized in that** a cosmetic composition as defined in Claims 1 to 6 is used.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3878537 A
- DD 274357
- US 5153230 A

**Littérature non-brevet citée dans la description**

- **ICHIHARA, GREENBERG.** *J. Biol. Chem.,* 1957, vol. 225, 945-958
- **MINOLTA.** *Analyse de couleurs - contrôle de couleurs : de la perception à l'instrumentation,* Janvier 1989